(19) **Europäisches Patentamt / European Patent Office / Office européen des brevets**

(11) **EP 3 992 305 A1**

(12) **EUROPEAN PATENT APPLICATION**
published in accordance with Art. 153(4) EPC

(43) Date of publication:
**04.05.2022 Bulletin 2022/18**

(21) Application number: **20815428.6**

(22) Date of filing: **28.05.2020**

(51) International Patent Classification (IPC):
***C12Q 1/6876*** *(2018.01)*

(52) Cooperative Patent Classification (CPC):
**C12Q 1/6816; C12Q 1/6876**

(86) International application number:
**PCT/KR2020/006934**

(87) International publication number:
**WO 2020/242226 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2019 KR 20190062639**

(71) Applicant: **PROGENEER INC.**
**Guro-gu**
**Seoul 08380 (KR)**

(72) Inventors:
- **UM, Soong Ho**
  **Seoul 06670 (KR)**
- **AHN, So Yeon**
  **Seoul 08011 (KR)**
- **SHIN, Seung Won**
  **Suwon-si Gyeonggi-do 16546 (KR)**

(74) Representative: **Budde Schou A/S**
**Dronningens Tvaergade 30**
**1302 Copenhagen K (DK)**

(54) **MULTIVALENT NUCLEIC ACID NANOSTRUCTURE FOR NUCLEIC ACID DETECTION, AND HIGHLY SENSITIVE NUCLEIC ACID PROBE USING SAME**

(57) The present invention relates to a nucleic acid probe system for increasing the detection efficiency of nucleic acid biomarkers, and improves the detection efficiency and detection sensitivity of biomarkers through a method for integrating a single-stranded probe sequence into a nucleic acid nanostructure. A nucleic acid nanostructure of the present invention, developed for this, converts, to a fast reaction inside the structure, some collision/reaction steps between molecules according to simple diffusion through localized aggregation of probe sequences, activates a fast signal amplification mechanism in the structure through structural flexibility so as to improve detection ability, thereby having effects of improving detection ability and effectively lowering a detection limit.

[Fig. 1]

<110>     Progeneer Inc.

<120>     Multivalent Nucleic Acid Nanostructure for Nucleic Acids Detection
and High-sensitive Nucleic Acid Probing Using The Same

<130>     PP-B2393

<150>     KR 2019-0062639
<151>     2019-05-28

<160>     21

<170>     KoPatentIn 3.0

<210>     1
<211>     36
<212>     DNA
<213>     Artificial Sequence

<220>
<223>     S1

<400>     1
cgaccgatga atagcggtca gatccgtacc tactcg                                    36

<210>     2
<211>     36
<212>     DNA
<213>     Artificial Sequence

<220>
<223>     S2

<400>     2
cgagtaggta cggatctgcg tattgcgaac gactcg                                    36

<210>     3
<211>     36
<212>     DNA
<213>     Artificial Sequence

<220>
<223>     S3

<400>     3
cgagtcgttc gcaatacggc tgtacgtatg gtctcg                                    36

<210>     4
<211>     36
<212>     DNA
<213>     Artificial Sequence

<220>
<223>     S4

<400>     4

cgagaccata cgtacagcac cgctattcat cggtcg                                    36


<210>     5
<211>     36
<212>     DNA
<213>     Artificial Sequence

<220>
<223>     S5


<400>     5
cgagaccata cgtacagcgc gatgcgcacg cgcacg                                    36


<210>     6
<211>     36
<212>     DNA
<213>     Artificial Sequence

<220>
<223>     S6


<400>     6
cgtgcgcgtg cgcatcgctg cggtgccgtg tgcacg                                    36


<210>     7
<211>     36
<212>     DNA
<213>     Artificial Sequence

<220>
<223>     S7


<400>     7
cgtgcacacg gcaccgcaac cgctattcat cggtcg                                    36


<210>     8
<211>     36
<212>     DNA
<213>     Artificial Sequence

<220>
<223>     S8


<400>     8
cgagtaggta cggatctgac cgctattcat cggtcg                                    36


<210>     9
<211>     36
<212>     DNA
<213>     Artificial Sequence

<220>
<223>     S9

```
<400>     9
cgagtcgttc gcaatacgac cgctattcat cggtcg                              36


<210>     10
<211>     83
<212>     DNA
<213>     Artificial Sequence

<220>
<223>     S1H1


<400>     10
cgaccgatga atagcggtca gatccgtacc tactcgtcaa catcagtctg ataagctacc    60

atgtgtagat agcttatcag act                                            83


<210>     11
<211>     79
<212>     DNA
<213>     Artificial Sequence

<220>
<223>     S2H2


<400>     11
cgagtaggta cggatctgcg tattgcgaac gactcgtaag ctatctacac atggtagctt    60

atcagactcc atgtgtaga                                                 79


<210>     12
<211>     83
<212>     DNA
<213>     Artificial Sequence

<220>
<223>     S3H1


<400>     12
cgagtcgttc gcaatacggc tgtacgtatg gtctcgtcaa catcagtctg ataagctacc    60

atgtgtagat agcttatcag act                                            83


<210>     13
<211>     79
<212>     DNA
<213>     Artificial Sequence

<220>
<223>     S4H2


<400>     13
cgagaccata cgtacagcac cgctattcat cggtcgtaag ctatctacac atggtagctt    60

atcagactcc atgtgtaga                                                 79
```

```
<210>    14
<211>    79
<212>    DNA
<213>    Artificial Sequence

<220>
<223>    S5H2


<400>    14
cgagaccata cgtacagcgc gatgcgcacg cgcacgtaag ctatctacac atggtagctt        60

atcagactcc atgtgtaga                                                      79


<210>    15
<211>    83
<212>    DNA
<213>    Artificial Sequence

<220>
<223>    S6H1


<400>    15
cgtgcgcgtg cgcatcgctg cggtgccgtg tgcacgtcaa catcagtctg ataagctacc        60

atgtgtagat agcttatcag act                                                 83


<210>    16
<211>    79
<212>    DNA
<213>    Artificial Sequence

<220>
<223>    S7H2


<400>    16
cgtgcacacg gcaccgcaac cgctattcat cggtcgtaag ctatctacac atggtagctt        60

atcagactcc atgtgtaga                                                      79


<210>    17
<211>    79
<212>    DNA
<213>    Artificial Sequence

<220>
<223>    S8H2


<400>    17
cgagtaggta cggatctgac cgctattcat cggtcgtaag ctatctacac atggtagctt        60

atcagactcc atgtgtaga                                                      79


<210>    18
```

```
<211>      79
<212>      DNA
<213>      Artificial Sequence

<220>
<223>      S9H2


<400>      18
cgagtcgttc gcaatacgac cgctattcat cggtcgtaag ctatctacac atggtagctt      60

atcagactcc atgtgtaga                                                    79


<210>      19
<211>      22
<212>      RNA
<213>      Artificial Sequence

<220>
<223>      mir-21


<400>      19
uagcuuauca gacugauguu ga                                                22


<210>      20
<211>      47
<212>      DNA
<213>      Artificial Sequence

<220>
<223>      H1


<400>      20
tcaacatcag tctgataagc taccatgtgt agatagctta tcagact                    47


<210>      21
<211>      43
<212>      DNA
<213>      Artificial Sequence

<220>
<223>      H2


<400>      21
taagctatct acacatggta gcttatcaga ctccatgtgt aga                        43
```

## Description

[Technical Field]

The present invention relates to a nucleic acid probe system for increasing the detection efficiency of a nucleic acid biomarker, and particularly to an improvement in the detection efficiency and detection sensitivity of a biomarker, achieved through a method of integrating a single-stranded probe sequence into a nucleic acid nanostructure.

[Background Art]

Methods of detecting specific nucleic acids (DNA or RNA) or proteins are fundamentally important techniques in the field of scientific research. Owing to the ability to detect and identify specific nucleic acids or proteins, researchers have been able to determine which genetic or biological markers are indicative of a person's health status. Methods of detecting nucleic acids and proteins enable detection of a modification of a pathogenic gene present in a sample or expression of a specific gene. Such molecular diagnosis serves to diagnose the root cause of a disease, such as DNA or RNA, and is employed in various fields such as those pertaining to infectious diseases, cancer diagnosis, genetic diseases, and personalized diagnosis. Molecular diagnostic technology is typically exemplified by a PCR technique for amplifying DNA within a short time (Saiki R. et. al., Science 239: 487-91., 1998). However, a general PCR technique has to use an electrophoresis method in order to identify the amplified DNA. Here, identifying DNA using electrophoresis is troublesome because an agarose gel must be made, DNA must be stained with EtBr, and so on. In addition, a recently used real-time PCR method does not require electrophoresis because it uses fluorescence, but is problematic in that expensive instruments and expensive fluorescent reagents have to be used (Higuchi R. et. al., Nature Biotechnology 11:1026-1030, 1993). Recently, Cepheid's GeneXpert systems and reagents, which are PCR products for point-of-care use, have been developed and sold, but the use thereof in general tests is difficult because the systems and reagents are very expensive (Helb D. et. al., J. Clin. Microbiol. 48:229-237, 2010). Another identification method is nucleic acid lateral flow assay, which uses a membrane instead of gel electrophoresis after PCR (Aveyard J. et. al., Chem. Commun., 41:4251-4253, 2007). However, this method is complicated compared to gel electrophoresis technology, so manufacture and use thereof in a laboratory become impossible, and general use thereof is limited due to the technical limitation in which the sequence of the probe attached to the membrane has to be used so as to specifically bind to the PCR amplification product. In particular, this PCR-based sequencing method is extremely vulnerable to specific errors and has problems related to data interpretation, limiting the accuracy and sensitivity of nucleic acid and protein detection.

Nucleic acid biomarkers separated and purified from cells, tissues, or blood for clinical diagnostic purposes are present at a low concentration, so signal amplification in the detection process is inevitable. Signal amplification techniques applied to nucleic acid biosensors comprise typical methods such as HCR (hybridization chain reaction) and RCA (rolling circle amplification), and such a conventional amplification method focuses on amplifying a signal generated after a probe recognizes a target. However, biosensors using this technology are essentially subjected to a process in which a reaction is initiated after a probe recognizes a target at an early stage, and this process is affected by the diffusion and collision of probes and target molecules. In particular, when the concentration of the target molecule is low, the collision frequency is drastically reduced, thereby blocking the reaction or creating noise in the signal amplification step. In this case, there is a problem in that the efficiency of target detection is lowered despite the subsequent amplification process.

For example, probe systems having excellent detection efficiency, such as HPA (hybridization protection assay), SDA (strand displacement amplification), TMA (transcription-mediated amplification), and DKA (dual kinetic assay), have been proposed in existing literature, and these were developed with the main focus of increasing the amplification efficiency of a signal generated after target recognition. However, even in systems having excellent signal amplification efficiency, when the concentration of biomarkers is very low, the intermolecular collision/reaction frequencies for target detection and signal amplification also decrease, which undesirably determines the reactivity of the probe system and the limit of detection sensitivity, and no solution therefor has been proposed.

Accordingly, the present inventors made great efforts to fundamentally improve the reactivity of probe systems and limits of detection sensitivity of biomarkers, developed a novel nucleic acid nanostructure in which single-stranded probe sequences are integrated in a multivalent form, and ascertained that the detection efficiency of biomarkers is improved by increasing the collision frequency between probes based on the flexibility of the nanostructure and the integration of the probes due to the formation of the nanostructure, thus culminating in the present invention.

[Disclosure]

It is an object of the present invention to provide a nucleic acid nanostructure comprising a probe pair complementary to a target nucleic acid.

It is another object of the present invention to provide a composition for detecting a nucleic acid comprising the nucleic acid nanostructure of the present invention.

It is still another object of the present invention to provide a method of preparing the nucleic acid nanostructure of the present invention.

It is yet another object of the present invention to provide a method of detecting a nucleic acid using the nucleic acid nanostructure of the present invention.

In order to accomplish the above objects, the present invention provides a nucleic acid nanostructure, in which probe-structure strands are hybridized with each other through complementary binding between structure nucleic acids, wherein each of the probe structure strands is formed by linking a probe pair, having hairpin loop and comprising a nucleotide sequence specifically binding to a target nucleic acid, with the structure nucleic acids comprising sequences complementary to each other.

The present invention provides a composition for detecting a target nucleic acid comprising the nucleic acid nanostructure.

In addition, the present invention provides a diagnostic kit comprising the composition for detecting a nucleic acid.

In addition, the present invention provides a method of preparing a nucleic acid nanostructure, comprising:

(a) constructing probe-structure strands in which a probe pair having a hairpin loop and comprising a nucleotide sequence specifically binding to a target nucleic acid is respectively linked with structure nucleic acids comprising sequences complementary to each other, and
(b) annealing the probe-structure strands to obtain a nucleic acid nanostructure.

In addition, the present invention provides a method of detecting a target nucleic acid using the nucleic acid nanostructure.

[Description of Drawing]

FIG. 1 shows (a) a schematic view of the process of reaction of a miR-21 amplification detection probe system, (b) a schematic view of the process of reaction of a probe system integrated into a DNA nanostructure, and (c) collision frequency and reactivity of DNA nanostructure probe systems;

FIG. 2 shows the results of electrophoretic analysis on the synthesis results of DNA nanostructures comprising H1 and H2 motifs,

(a) showing the result of 1× TBE 12% polyacrylamide gel electrophoresis of D-DNA,
(b) showing the result of 1× TBE 5% polyacrylamide gel electrophoresis of T-DNA,
(c) showing the result of 1× TBE 12% polyacrylamide gel electrophoresis of Y-DNA, and
(d) showing the result of 1× TBE 5% polyacrylamide gel electrophoresis of H-DNA;

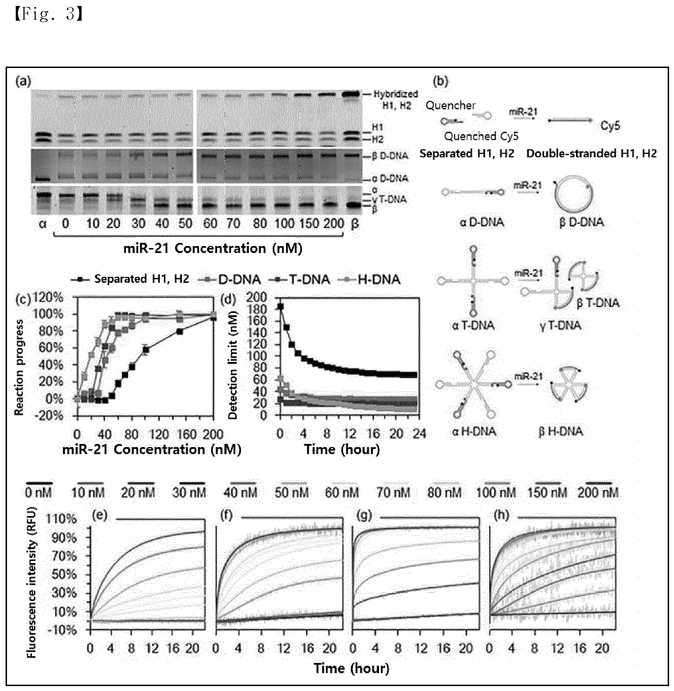

FIG. 3 shows (a) the results of electrophoretic analysis on the reaction of separated H1 and H2, D-DNA, T-DNA and H-DNA with miR-21 at various concentrations, in which H1 and H2 motifs have the same concentration (100 nM), (b) schematic views (structural changes depending on intermolecular and intramolecular reactions with miR-21), (c) the extent of reaction depending on the concentration of miR-21 24 hours after reaction, and (d) the detection limit depending on the reaction time,

(e) showing the time-dependent reaction of separated H1 and H2 with miR-21 at various concentrations,
(f) showing the time-dependent reaction of D-DNA with miR-21 at various concentrations,
(g) showing the time-dependent reaction of T-DNA with miR-21 at various concentrations,
(h) showing the time-dependent reaction of H-DNA with miR-21 at various concentrations,

α indicating the state in which a DNA nanostructure is synthesized through dual annealing and H1 and H2 are not double-stranded, and
β indicating a DNA nanostructure state in which H1 and H2 are double-stranded through general annealing;

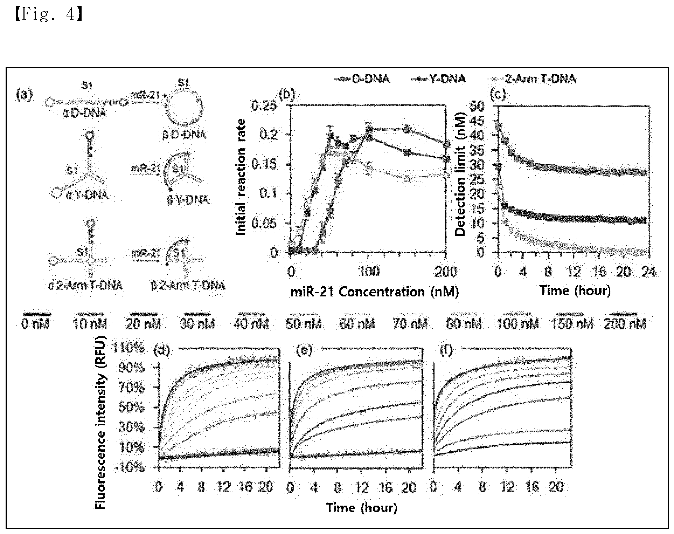

FIG. 4 shows (a) the results of structural changes of D-DNA, Y-DNA, and 2-arm T-DNA depending on the intramolecular reaction with miR-21 and location information of fluorescent dyes and quenchers for real-time reaction measurement, (b) the initial reaction rate of each probe system at various miR-21 concentrations, (c) the detection limit depending on the reaction time of each probe system, and the time-dependent reaction of D-DNA (d), Y-DNA (e), and 2-arm T-DNA (f) with miR-21 at various concentrations;

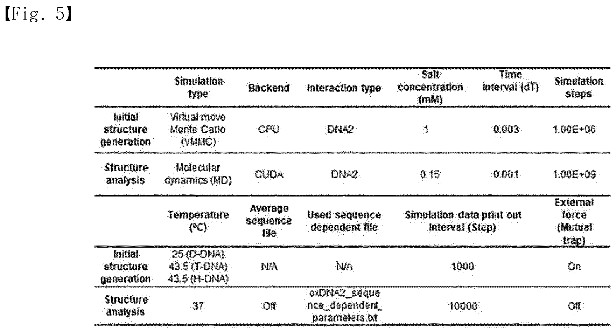

FIG. 5 shows the parameters of the oxDNA nucleic acid simulation program used for the formation of a DNA nanostructure and the analysis of the distance between paired bases in the structure;

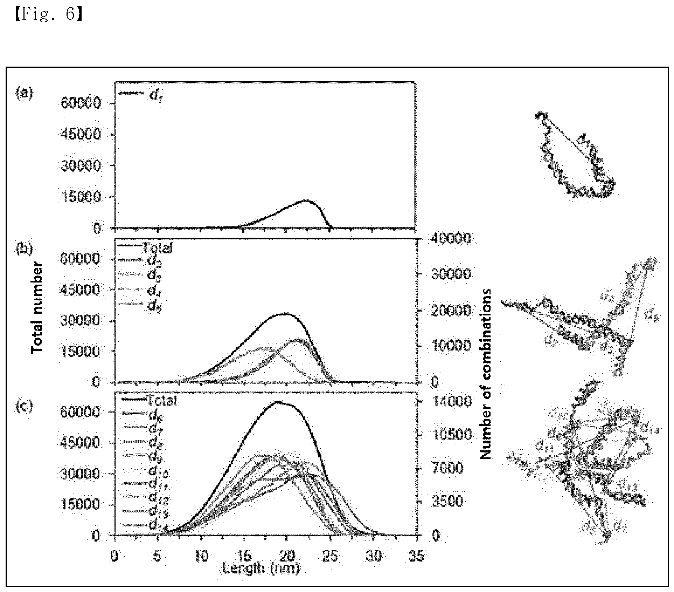

FIG. 6 shows the distances between the molecular H1 and H2 motifs in D-DNA (a), T-DNA (b), and H-DNA (c) through oxDNA simulation analysis;

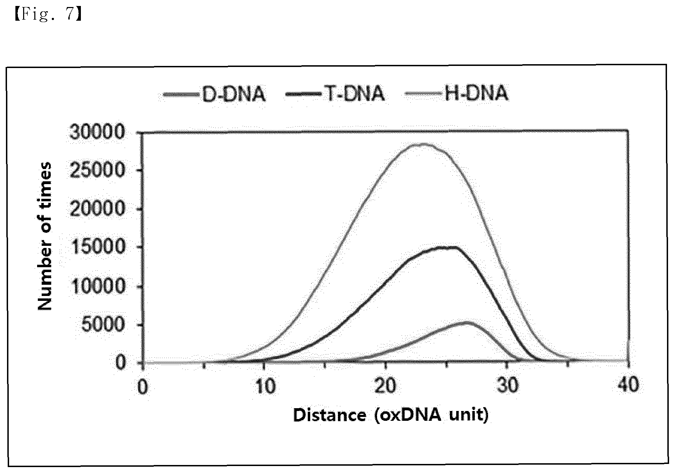

FIG. 7 shows the number of times the distance between H1 and H2 motifs in D-DNA, T-DNA, and H-DNA reaches a specific distance; and

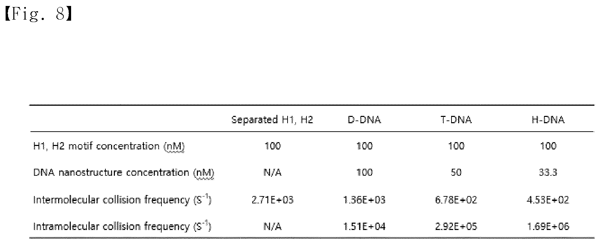

FIG. 8 shows the collision frequency of intermolecular reactions of H1 and H2 motifs of each DNA nanostructure calculated using a Smoluchowski equation and the collision frequency of intramolecular reactions of H1 and H2 motifs of each DNA nanostructure inferred through oxDNA simulation analysis.

[Mode for Invention]

Unless otherwise defined, all technical and scientific terms used herein have the same meanings as those typically understood by those skilled in the art to which the present invention belongs. Generally, the nomenclature used herein is well known in the art and is typical.

Unless otherwise indicated, nucleic acids are set forth in a 5' → 3' direction from left to right. Numerical ranges recited within the specification are inclusive of the numbers defining the range, comprising each integer or any non-integer fraction within the defined range.

Although any methods and materials similar or equivalent to those described herein may be used in practice to test the present invention, the preferred materials and methods are described herein.

The present invention is intended to develop a highly sensitive probe system for detecting a target nucleic acid, and when a novel nucleic acid nanostructure comprising multivalent single-stranded probe sequences is constructed and used to detect a target nucleic acid, it can be confirmed that the detection efficiency of biomarkers is improved by increasing the collision frequency between probes based on the flexibility of the nanostructure.

An aspect of the present invention pertains to a nucleic acid nanostructure, in which probe-structure strands are hybridized with each other through complementary binding between structure nucleic acids, wherein each of the probe structure strands is formed by linking a probe pair, having hairpin loop and comprising a nucleotide sequence specifically binding to a target nucleic acid, with the structure nucleic acids comprising sequences complementary to each other.

As used herein, the term "nucleic acid nanostructure" comprises structures comprising a probe pair comprising a sequence complementary to a target nucleic acid, a structure nucleic acid, a fluorophore, and a quencher. In the present invention, the nucleic acid nanostructure may be of a dimer type (D type), Y type, tetramer type (T type), or hexamer type (H type), but is not limited thereto.

As used herein, the term "probe" refers to a nucleic acid fragment, such as RNA or DNA, having a length corresponding to ones of bases to hundreds of bases, capable of specifically binding to a nucleic acid (e.g. a biomarker) comprising a specific sequence, and the probe is labeled such that the presence or absence of a specific nucleic acid may be identified. The probe may be constructed in the form of an oligonucleotide probe, a single-stranded DNA probe, an RNA probe, or the like.

In the present invention, the probe pair is a pair of single-stranded probes having a hairpin loop, and in the present specification, these probes are referred to as a "first probe" and a "second probe" to distinguish therebetween. In part of the present specification, for convenience of description, a probe that specifically binds to a target nucleic acid is referred to as a "first probe" and a probe that binds to the first probe through an immediate intramolecular reaction is referred to as a "second probe", which may be used by changing the order of ordinal numbers. Ordinal numbers such as "first" and "second" are used to distinguish probes constituting the probe pair, do not limit the scope of rights, and are used in the same way to distinguish "structure nucleic acids".

In the present invention, at least one probe of the probe pair may comprise a nucleotide sequence that specifically binds to a complementary target nucleic acid.

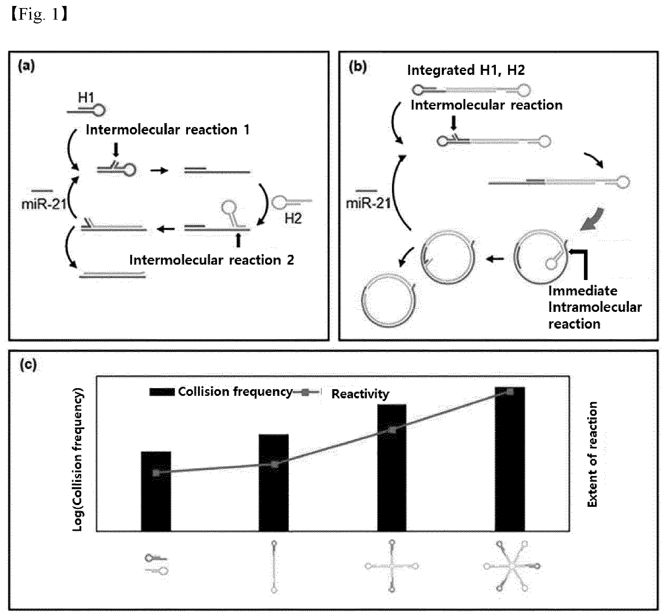

In the present invention, the probes of the probe pair may comprise sequences complementary to each other. Preferably, for immediate reaction between the probes, part of the sequence other than the hairpin loop of one probe is complementary to part of the sequence of the remaining probe, and more preferably, the part of the sequence that is exposed as a single strand when the hairpin loop of one probe is unwound is complementary to part of the sequence of the remaining probe (FIGS. 1 and 3) .

As used herein, the term "structure nucleic acid" refers to an oligonucleic acid that induces the formation of a multivalent nucleic acid nanostructure by integrating the probes.

In the present invention, since the structure nucleic acids comprise sequences complementary to each other in parts thereof, a nucleic acid nanostructure may be formed through hybridization therebetween.

In the present invention, when there are three or more structure nucleic acids, each structure nucleic acid may comprise a sequence complementary to partial sequences of one or more other structure nucleic acids, so a nucleic acid nanostructure may be formed through hybridization therebetween.

In the present invention, the structure nucleic acid may be used as it is, or may be used in the form of a probe-structure strand by being linked to a probe. The structure nucleic acid may comprise a sequence complementary to all or part of two or more other structure nucleic acids, and the form of the nucleic acid nanostructure may be changed depending on the combination thereof. In an embodiment of the present invention, structure nucleic acids having the sequences of SEQ ID NO: 1 to SEQ ID NO: 9 are used, but the present invention is not limited thereto.

As used herein, the term "link" in the context of link of the 'probe' and 'structure nucleic acid' or link of the 'probe or structure nucleic acid' and the 'quencher or fluorophore' means that the probe, the structure, the quencher, and the fluorophore are linked in a way that may be easily grasped by those of ordinary skill in the art. The "link" may directly linked using, for example, a hydrogen bond, a covalent bond, an electrical bond, a van der Waals bond, or the like, or may indirectly linked using a linker or the like. In an embodiment of the present invention, the probe and the structure nucleic acid are linked in a manner in which the 5' end of the probe and the 3' end of the structure nucleic acid are linked to each other using a phosphate diester bond and the probe or the structure nucleic acid is linked to the quencher or the fluorophore using a covalent bond, but the present invention is not limited thereto.

As used herein, the term "complementary" is used to describe the relationship between nucleotide bases capable of hybridizing with each other. For example, in Watson-Crick base pairing of DNA, adenosine is complementary to thymine and cytosine is complementary to guanine. Accordingly, the present invention also comprises sequences that are "complementary" due to being substantially similar nucleic acid sequences, as well as fully complementary sequences disclosed or used herein.

In the present invention, the "sequences complementary to each other" used in the "probe" are independent of the "sequences complementary to each other" used in the "structure nucleic acid".

In the present invention, when any one of the two single-stranded probes constituting the probe pair binds to the target nucleic acid, the hairpin loop thereof may be unwound.

In the present invention, after the hairpin loop of the probe bound to the target nucleic acid is unwound, it may complementarily bind to the remaining probe of the probe pair through an immediate intramolecular reaction.

In the present invention, when the probe bound to the target nucleic acid complementarily binds to the remaining probe of the probe pair through an immediate intramolecular reaction, the hairpin loop of the remaining probe may be unwound.

As used herein, the term "immediate intramolecular reaction" refers to a reaction that is able to occur when the first probe and the second probe form a bond through a complementary sequence, and due to the formation of the nucleic acid nanostructure of the present invention, the immediate intramolecular reaction may exhibit much higher reactivity than the reaction of individual probe molecules. This reaction is illustrated in detail in FIGS. 1b, 3b and 4a according to each embodiment.

In the present invention, when two probes constituting the probe pair complementarily bind to each other, the target nucleic acid may be separated, and may react with other nucleic acid nanostructures.

In the present invention, even when the target nucleic acid is separated due to binding of the first probe and the second probe, the formation of a hairpin loop is suppressed to thereby continuously maintain the shape of the linear probe and generate a detection signal (e.g. fluorescence).

The nucleic acid nanostructure according to the present invention comprises multivalent probe sequences in one structure, so probes capable of binding to a target nucleic acid are spatially integrated, and the number of branches and flexibility thereof are high, whereby accessibility and collision frequency between the probes are high, and thus sensitivity to the target nucleic acid is increased and the detection limit is decreased.

As used herein, the term "target nucleic acid" refers to a nucleic acid that is a target to be detected. The target nucleic acid is preferably single-stranded, and may be, for example, a short RNA fragment such as miRNA, siRNA, piwiRNA, snoRNA, etc., but is not limited thereto. In the present invention, the "target nucleic acid" is preferably a nucleotide sequence of 10 to 40 bp, but is not limited thereto.

In one embodiment, the nucleic acid nanostructure may comprise a structure nucleic acid in which a probe is linked to the 3' end thereof.

In one embodiment, the nucleic acid nanostructure may comprise one pair, two pairs, or three pairs of probes in one nanostructure. Examples of the structure comprising one pair of probes may comprise a dimer structure comprising respective single-stranded probes of a probe pair at both ends of a straight line, a Y-type structure comprising respective single-stranded probes of a probe pair at the ends of two branches, among three double-stranded branches, and a 2-arm tetramer structure comprising respective single-stranded probes of a probe pair at the ends of two branches, among four double-stranded branches forming a cross shape; and the nucleic acid nanostructure comprising two pairs of probes in one structure may be a tetramer structure comprising probes at the ends of four double-stranded branches; and the nucleic acid nanostructure comprising three pairs of probes in one structure may be a hexamer structure comprising single-stranded probes of each of probe pairs alternating at the ends of six double-stranded branches (FIGS. 3b and 4a).

For the pairs of probes of the present invention, the same probe pair is preferably used in one pair, two pairs, or three pairs, but the present invention is not limited thereto, and different probe pairs that specifically bind to the same or different target nucleic acid may be used in combination.

In the present invention, the nucleic acid nanostructure comprising one pair of probes may be a dimer-type (D-type) structure comprising respective nucleic-acid structure model probes of a probe pair at both ends of a straight line.

In the present invention, the dimer-type (D-type) structure may be consisted of a first probe-first structure strand and a second probe-second structure strand, and wherein the first structure strand and the second structure strand comprising complementary sequences may be complementarily bound to each other.

In the present invention, the nucleic acid nanostructure comprising one pair of probes may be a Y-type structure comprising respective single-stranded probes of a probe pair at the ends of two branches, among three double-stranded branches.

In the present invention, the Y-type structure may be consisted of a first probe-first structure strand, a second structure strand, and a second probe-third structure strand,

wherein the first structure nucleic acid may comprise a sequence complementary to a partial sequence of the second structure nucleic acid and a sequence complementary to a partial sequence of the third structure nucleic acid, the second structure nucleic acid may comprise a sequence complementary to a partial sequence of the first structure nucleic acid and a sequence complementary to a partial sequence of the third structure nucleic acid, and the third structure nucleic acid may comprise a sequence complementary to a partial sequence of the first structure nucleic acid and a sequence complementary to a partial sequence of the second structure nucleic acid.

Accordingly, the first probe-first structure strand, the second structure strand, and the second probe-third structure strand may be complementarily bound to each other.

In the present invention, the nucleic acid nanostructure comprising one pair of probes may be a 2-arm tetramer structure comprising respective single-stranded probes of a probe pair at the ends of two branches, among four double-stranded branches forming a cross shape.

In the present invention, the 2-arm tetramer structure may be consisted of a first probe-first structure strand, a second structure strand, a third structure strand, and a second probe-fourth structure strand, and
wherein the first structure nucleic acid may comprise a sequence complementary to a partial sequence of the second structure nucleic acid and a sequence complementary to a partial sequence of the fourth structure nucleic acid, the second structure nucleic acid may comprise a sequence complementary to a partial sequence of the first structure nucleic acid and a sequence complementary to a partial sequence of the third structure nucleic acid, the third structure nucleic acid may comprise a sequence complementary to a partial sequence of the second structure nucleic acid and a sequence complementary to a partial sequence of the fourth structure nucleic acid, and the fourth structure nucleic acid may comprise a sequence complementary to a partial sequence of the third structure nucleic acid and a sequence complementary to a partial sequence of the first structure nucleic acid.

Accordingly, the first probe-first structure strand, the second structure strand, the third structure strand, and the second probe-fourth structure strand may be complementarily bound to each other.

In the present invention, the nucleic acid nanostructure comprising two pairs of probes may be a tetramer-type (T-type) structure comprising single-stranded probes of each of two probe pairs at the ends of four double-stranded branches.

In the present invention, the tetramer-type (T-type) structure may be consisted of a first probe-first structure strand, a second probe-second structure strand, a first probe-third structure strand, and a second probe-fourth structure strand, and
wherein the first structure nucleic acid may comprise a sequence complementary to a partial sequence of the second structure nucleic acid and a sequence complementary to a partial sequence of the fourth structure nucleic acid, the second structure nucleic acid may comprise a sequence complementary to a partial sequence of the first structure nucleic acid and a sequence complementary to the third structure nucleic acid, the third structure nucleic acid may comprise a sequence complementary to a partial sequence of the second structure nucleic acid and a sequence complementary to the fourth structure nucleic acid, and the fourth structure nucleic acid may comprise a sequence complementary to a partial sequence of the first structure nucleic acid and a sequence complementary to a partial sequence of the third structure nucleic acid.

Accordingly, the first probe-first structure strand, the second probe-second structure strand, the first probe-third structure strand, and the second probe-fourth structure strand may be complementarily bound to each other.

In the present invention, the nucleic acid nanostructure comprising three pairs of probes may be a hexamer-type (H-type) structure comprising single-stranded probes of each of probe pairs alternating at the ends of six double-stranded branches.

In the present invention, the hexamer-type (H-type) structure may be consisted of a first probe-first structure strand, a second probe-second structure strand, a first probe-third structure strand, a second probe-fourth structure strand, a first probe-fifth structure strand, and a second probe-sixth structure strand, and

wherein the first structure nucleic acid may comprise a sequence complementary to a partial sequence of the second structure nucleic acid and a sequence complementary to a partial sequence of the sixth structure nucleic acid, the second structure nucleic acid may comprise a sequence complementary to a partial sequence of the first structure nucleic acid and a sequence complementary to a partial sequence of the third structure nucleic acid, the third structure nucleic acid may comprise a sequence complementary to a partial sequence of the second structure nucleic acid and a sequence complementary to a partial sequence of the fourth structure nucleic acid, the fourth structure nucleic acid may comprise a sequence complementary to a partial sequence of the third structure nucleic acid and a sequence complementary to a partial sequence of the fifth structure nucleic acid, the fifth structure nucleic acid may comprise a sequence complementary to a partial sequence of the fourth structure nucleic acid and a sequence complementary to a partial sequence of the sixth structure nucleic acid, and the sixth structure nucleic acid may comprise a sequence complementary to a partial sequence of the first structure nucleic acid and a sequence complementary to a partial sequence of the fifth structure nucleic acid.

Accordingly, the first probe-first structure strand, the second probe-second structure strand, the first probe-third structure strand, the second probe-fourth structure strand, the first probe-fifth structure strand, and the second probe-sixth structure strand may be complementarily bound to each other.

In one embodiment, the structure nucleic acid having a complementary configuration linked to each probe may be comprised, the structure nucleic acid linked to each probe having a hairpin loop may comprise a complementary sequence, and a fluorophore or a quencher may be linked to the end of each of the probe and the structure single-stranded nucleic acid.

In an embodiment of the present invention, for the D-structure, a quencher is attached to the end of the first probe and a fluorophore is attached to the end of the second structure single-stranded nucleic acid, so the first probe and the first structure single-stranded nucleic acid are complementarily bound to the second probe and the second structure single-stranded nucleic acid. When the first probe has a hairpin loop, no significant fluorescence signal is detected (αD-DNA in FIG. 3b). Here, the first probe in the state in which the first structure single-stranded nucleic acid and the second structure single-stranded nucleic acid are complementarily bound to each other is subjected to an intramolecular reaction with the target nucleic acid sequence to thus change the configuration, whereby the quencher and the fluorophore become farther away, thus emitting a fluorescent signal (βB-DNA of FIG. 3b).

In one embodiment, the nucleic acid nanostructure (dimer structure) comprising one pair of probes in one structure may comprise a single-stranded nucleic acid comprising a first structure nucleic acid linked to the first probe of a probe pair; and a single-stranded nucleic acid comprising a second structure nucleic acid linked to the second probe of the probe pair, wherein the first structure nucleic acid and the second structure nucleic acid may be complementary to each other.

In one embodiment, the nucleic acid nanostructure (Y-type structure) comprising one pair of probes in one structure may comprise a single-stranded nucleic acid comprising a first structure nucleic acid linked to the first probe of a probe pair; a single-stranded nucleic acid comprising a second structure nucleic acid; and a third structure nucleic acid linked to the second probe of the probe pair, wherein the first structure nucleic acid may be complementary to partial sequences of the second structure nucleic acid and the third structure nucleic acid, the second structure nucleic acid may be complementary to partial sequences of the first structure nucleic acid and the third structure nucleic acid, and the third structure nucleic acid may be complementary to partial sequences of the first structure nucleic acid and the second structure nucleic acid.

In one embodiment, the nucleic acid nanostructure (2-arm T-structure) comprising one pair of probes in one structure may comprise a single-stranded nucleic acid comprising a first structure nucleic acid linked to the first probe of a probe pair; a single-stranded nucleic acid comprising a second structure nucleic acid; a third structure nucleic acid; and a fourth structure nucleic acid linked to the second probe of the probe pair, wherein the first structure nucleic acid may be complementary to partial sequences of the second structure nucleic acid and the fourth structure nucleic acid, the second structure nucleic acid may be complementary to partial sequences of the first structure nucleic acid and the third structure nucleic acid, the third structure nucleic acid may be complementary to partial sequences of the second structure nucleic acid and the fourth structure nucleic acid, and the fourth structure nucleic acid may be complementary to partial sequences of the first structure nucleic acid and the third structure nucleic acid.

In one embodiment, the nucleic acid nanostructure (T-structure) comprising two pairs of probes in one structure may comprise a single-stranded nucleic acid comprising a first structure nucleic acid linked to the first probe of a probe pair; a single-stranded nucleic acid comprising a second structure nucleic acid linked to the second probe of the probe pair; a third structure nucleic acid linked to the first probe of another probe pair; and a fourth structure nucleic acid linked to the second probe of the probe pair, wherein the first structure nucleic acid may be complementary to partial sequences of the second structure nucleic acid and the fourth structure nucleic acid, the second structure nucleic acid may be complementary to partial sequences of the first structure nucleic acid and the third structure nucleic acid, the third structure nucleic acid may be complementary to partial sequences of the second structure nucleic acid and the fourth structure nucleic acid, and the fourth structure nucleic acid may be complementary to partial sequences of the first structure nucleic acid and the third structure nucleic acid.

In one embodiment, the nucleic acid nanostructure (H-structure) comprising three pairs of probes in one structure may comprise a single-stranded nucleic acid comprising a first structure nucleic acid linked to the first probe of a probe pair; a single-stranded nucleic acid comprising a second structure nucleic acid linked to the second probe of the probe pair; a third structure nucleic acid linked to the first probe of another probe pair; a fourth structure nucleic acid linked to the second probe of the probe pair; a fifth structure nucleic acid linked to the first probe of a further probe pair; and a sixth structure nucleic acid linked to the second probe of the probe pair, wherein the first structure nucleic acid may be complementary to partial sequences of the second structure nucleic acid and the sixth structure nucleic acid, the second structure nucleic acid may be complementary to partial sequences of the first structure nucleic acid and the third structure nucleic acid, the third structure nucleic acid may be complementary to partial sequences of the second structure nucleic acid and the fourth structure nucleic acid, the fourth structure nucleic acid may be complementary to partial sequences of the third structure nucleic acid and the fifth structure nucleic acid, the fifth structure nucleic acid may be complementary to partial sequences of the fourth structure nucleic acid and the sixth structure nucleic acid, and the sixth structure nucleic acid may be complementary to partial sequences of the first structure nucleic acid and the fifth structure nucleic acid.

In one embodiment, the structure nucleic acid may comprise S1 comprising the nucleotide sequence of SEQ ID NO: 1, S2 comprising the nucleotide sequence of SEQ ID NO: 2, S3 comprising the nucleotide sequence of SEQ ID NO: 3, S4 comprising the nucleotide sequence of SEQ ID NO: 4, S5 comprising the nucleotide sequence of SEQ ID NO: 5, S6 comprising the nucleotide sequence of SEQ ID NO: 6, S7 comprising the nucleotide sequence of SEQ ID NO: 7, S8 comprising the nucleotide sequence of SEQ ID NO: 8, S9 comprising the nucleotide sequence of SEQ ID NO: 9, or combinations thereof. In an embodiment of the present invention, the D-structure comprises S1 and S8, the T-structure comprises S1, S2, S3, and S4, the H-structure comprises S1, S2, S3, S5, S6, and S7, the Y-structure comprises S1, S2, and S9, and the 2-arm T-structure comprises S1, S2, S3, and S4.

In the present invention, the nucleic acid nanostructure may comprise a quencher and a fluorophore.

In one embodiment, the nanostructure may comprise a quencher at the 5' end or 3' end thereof, and may comprise a fluorophore at the 3' end or 5' end thereof.

In one embodiment, the fluorophore may be selected from the group consisting of FAM, TET, HEX, Cy3, TMR, ROX, Texas red, Cy5, Cy 5.5, JOE, VIC, NED, CAL Fluor Orange 560, CAL Fluor Red 590, CAL Fluor Red 610, Quasar 570, Oyster 556, Oyster 645, LC red 640, LC red 670, LC red 705, and quantum dots.

In one embodiment, the quencher may be selected from the group consisting of DDQ-I, DDQ-II, DABCYL, Eclipse, Iowa black FQ, Iowa black RQ, BHQ-1, BHQ-2, BHQ-3, QSY-21, QSY-7, gold nanoparticles, carbon nanotubes, graphene, FAM, TET, HEX, Cy3, TMR, ROX, Texas red, Cy5, Cy 5.5, JOE, VIC, NED, CAL Fluor Orange 560, CAL Fluor Red 590, CAL Fluor Red 610, Quasar 570, Oyster 556, Oyster 645, LC red 640, LC red 670, LC red 705, and quantum dots.

In one embodiment, in the nucleic acid nanostructure comprising a probe pair complementary to a target nucleic acid of the present invention in one structure, the probe pair, which does not react with each other due to the hairpin loop, reacts sequentially due to the catalytic role of the target nucleic acid, and thus the hairpin loop is unwound and a double strand is formed through complementary binding, whereby the fluorophore quenched using each quencher is able to emit light.

In one embodiment, the nucleic acid may be DNA, RNA, or DNA and RNA.

In one embodiment, the probes of the present invention may be chemically synthesized using a phosphoramidite solid support method or other well-known methods. The nucleic acid sequences may also be modified using any means known in the art. Non-limiting examples of such modification comprise methylation, encapsulation, substitution of one or more native nucleotides with analogues thereof, and inter-nucleotide modifications, for example modifications to uncharged conjugates (e.g. methyl phosphonate, phosphotriester, phosphoramidate, carbamate, etc.) or to charged conjugates (e.g. phosphorothioate, phosphorodithioate, etc.).

The nucleic acid nanostructure of the present invention enables the reaction process to occur as an intramolecular reaction rather than an intermolecular reaction by integrating nucleic acid probes, which are a reactant of the detection process, into the nucleic acid nanostructure. Therefore, the reactant dispersed in the solution is integrated into one molecule, so a fast and efficient reaction is induced using a limited amount of catalyst, and some diffusion process of the reactant, which inhibits the reaction, is not carried out, causing high collision frequency between probes inside the nucleic acid structure, ultimately improving reactivity, diagnostic sensitivity, and detection speed.

In one embodiment, the nucleic acid nanostructure of the present invention may be a nucleic acid nanostructure comprising H1 (SEQ ID NO: 20) and H2 (SEQ ID NO: 21) as a probe pair for the target nucleic acid miR-21 (SEQ ID NO: 19) and comprising, as a structure nucleic acid, S1 (SEQ ID NO: 1), S2 (SEQ ID NO: 2), S3 (SEQ ID NO: 3), S4 (SEQ ID NO: 4), S5 (SEQ ID NO: 5), S6 (SEQ ID NO: 6), S7 (SEQ ID NO: 7), S8 (SEQ ID NO: 8), S9 (SEQ ID NO: 9), S1H1 (SEQ ID NO: 10), S2H2 (SEQ ID NO: 11), S3H1 (SEQ ID NO: 12), S4H2 (SEQ ID NO: 13), S5H2 (SEQ ID NO: 14), S6H1 (SEQ ID NO: 15), S7H2 (SEQ ID NO: 16), S8H2 (SEQ ID NO: 17), S9H2 (SEQ ID NO: 18), or combinations thereof.

Another aspect of the present invention pertains to a composition for detecting a nucleic acid comprising the nucleic acid nanostructure.

As used herein, the term "sample" comprises tissue, cell, blood, serum, urine, saliva, plasma or body fluid obtained from a subject or patient, and the source of the tissue or cell sample is fresh, frozen, and/or preserved organ or tissue samples or solid tissue from biopsies or aspirates, blood or any blood constituent, and cells at any point in the development or pregnancy of a subject. The tissue sample may also be a primary or cultured cell or cell line.

As used herein, the term "detection" or "measurement" means quantifying the concentration of a detected or measured target.

As used herein, the term "target nucleic acid" refers to a nucleic acid that is a target to be detected. The target nucleic acid is preferably single-stranded, for example, a short RNA fragment such as miRNA, siRNA, piwiRNA, snoRNA, etc., but is not limited thereto. In the present invention, the "target nucleic acid" is preferably a nucleotide sequence of 10 to 40 bp, but is not limited thereto.

Still another aspect of the present invention pertains to a diagnostic kit comprising the composition for detecting a target nucleic acid of the present invention.

Yet another aspect of the present invention pertains to a method of preparing a nucleic acid nanostructure, comprising:

(a) constructing probe-structure strands in which a probe pair having a hairpin loop and having a nucleotide sequence specifically binding to a target nucleic acid is respectively linked with structure nucleic acids comprising sequences complementary to each other, and
(b) annealing the probe-structure strands to obtain a nucleic acid nanostructure.

In one embodiment, the probe-structure strands may be constructed by linking the probes and the structure nucleic acids through annealing from 95°C to 4°C at a rate of -0.5 °C/30 seconds.

In one embodiment, each of the probe-structure strands may be set to a concentration of 500 to 700 nM, maintained at 40 to 45°C for 3 to 10 minutes, and then annealed to 4°C at a rate of -0.5 °C/30 seconds, thereby constructing a D-structure.

In one embodiment, each of the probe-structure strands may be set to a concentration of 800 to 1000 nM, maintained at 40 to 45°C for 3 to 10 minutes, and then annealed to 4°C at a rate of -0.5 °C/30 seconds, thereby constructing a Y-structure.

In one embodiment, each of the probe-structure strands may be set to a concentration of 1100 to 1300 nM, maintained at 40 to 45°C for 3 to 10 minutes, and then annealed to 4°C at a rate of -0.5 °C/30 seconds, thereby constructing a 2-arm T-structure or a T-structure.

In one embodiment, each of the structure nucleic acids linked to the probes may be set to a concentration of 1700 to 1900 nM, maintained at 50 to 60°C for 3 to 10 minutes, and then annealed to 4°C at a rate of - 0.1 °C/144 seconds, thereby constructing an H-structure.

In one embodiment, annealing may be performed two times in order to form a double-stranded branch of the structure while maintaining the state in which the hairpin loop of each probe single strand of the probe pair, which is part of the nucleic acid nanostructure, is not unwound or does not incidentally bind with other sequences. The single strands constituting the structure (two strands for the D-structure, four strands for the T-structure, six strands for the H-structure, three strands for the Y-structure, and four strands for the 2-arm T-structure) may be separately annealed, thus completing the hairpin loop of the probe single-stranded motif, after which the single strands may be annealed by setting the initial temperature to a low temperature so that the hairpin loop is not unwound.

Still yet another aspect of the present invention pertains to a method of detecting a nucleic acid using the nucleic acid nanostructure or the composition for detecting a nucleic acid.

Examples

A better understanding of the present invention may be obtained through the following examples. These examples are merely set forth to illustrate the present invention, and are not to be construed as limiting the scope of the present invention, as will be apparent to those of ordinary skill in the art.

Example 1. Synthesis of DNA nanostructure comprising detection probe pair

1-1. Synthesis of separated H1 and H2

Fluorescence signal amplification was induced through an isothermal strand displacement reaction of a probe pair H1 and H2 having a hairpin loop for detection of a target nucleic acid miR-21. Specifically, a sequence in which BHQ2 (black hole quencher 2) was substituted at the 3' end of the H1 sequence and a Cy5 fluorophore was substituted at the 5' end thereof was used so that the unwinding of the hairpin loop could be confirmed based on an increase in the fluorescence signal. 100 μL of a solution in which the concentration of the H1 sequence was 300 nM and the concentration of NaCl(aq) was 200 mM was prepared, maintained at 95°C for 5 minutes in a thermal cycler, and then annealed from 95°C to 4°C at a rate of -0.5 °C/30 seconds. The H2 sequence was also prepared in the same manner as above.

1-2. Synthesis of dimer-DNA (D-DNA) structure

A nanostructure in the form of a dimer (having two double-stranded branches and comprising one pair of H1 and H2) using a probe pair H1 and H2 having a hairpin loop for detection of a target nucleic acid miR-21 was synthesized. Specifically, sequences in which BHQ2 (black hole quencher 2) was substituted at the 3' end of the S1H1 sequence and a Cy5 fluorophore was substituted at the 5' end of the S8H2 sequence were used so that unwinding of the hairpin loop could be confirmed based on an increase in the fluorescence signal. 50 μL of each of solutions in which the concentration of the S1H1 or S8H2 sequence was 600 nM and the concentration of NaCl(aq) was 200 mM was prepared, maintained at 95°C for 5 minutes in a thermal cycler, and then annealed from 95°C to 4°C at a rate of -0.5 °C/30 seconds. Thereafter, 50 μL of each of the S1H1 and S8H2 solutions having a NaCl(aq) concentration of 200 mM and a sequence concentration of 600 nM was mixed, maintained at 43.5°C for 5 minutes in a thermal cycler, and annealed to 4°C at a rate of - 0.5 °C/30 seconds. Thereby, 100 μL of a D-DNA solution in which the final concentration of the structure was 300 nM and the final concentration of NaCl(aq) was 200 mM was completed.

1-3. Synthesis of tetramer-DNA (T-DNA) structure

A nanostructure in the form of a tetramer (having four double-stranded branches and comprising two pairs of H1 and H2) using a probe pair H1 and H2 having a hairpin loop for detection of a target nucleic acid miR-21 was synthesized. Specifically, sequences in which BHQ2 was substituted at the 3' end of each of the S1H1 sequence and the S3H1 sequence and a Cy5 fluorophore was substituted at the 5' end of each of the S2H2 and S4H2 sequences were used, and 25 μL of each of solutions in which the concentration of the S2H2, S3H1, S4H2 or S1H1 sequence was 1200 nM and the concentration of NaCl(aq) was 200 mM was prepared, maintained at 95°C for 5 minutes in a thermal cycler, and then annealed from 95°C to 4°C at a rate of -0.5 °C/30 seconds. Thereafter, 25 μL of each of the S1H1, S2H2, S3H1 and S4H2 solutions having a NaCl(aq) concentration of 200 mM and a sequence concentration of 1200 nM was mixed, maintained at 43.5°C for 5 minutes in a thermal cycler, and then annealed from 43.5°C to 4°C at a rate of -0.5 °C/30 seconds, thereby completing 100 μL of a T-DNA solution in which the final concentration of the structure was 300 nM and the final concentration of NaCl(aq) was 200 mM.

1-4. Synthesis of hexamer-DNA (H-DNA) structure

A nanostructure in the form of a hexamer (having six double-stranded branches and comprising three pairs of H1 and H2) using a probe pair H1 and H2 having a hairpin loop for detection of a target nucleic acid miR-21 was synthesized. Specifically, sequences in which BHQ2 was substituted at the 3' end of each of the S1H1 sequence, S3H1 sequence, and S6H1 sequence and a Cy5 fluorophore was substituted at the 5' end of each of the S2H2 sequence, S5H2 sequence, and S7H2 sequence were used. 16.7 μL of each of solutions in which the concentration of the S2H2, S3H1, S5H2, S6H1, S7H2 or S1H1 sequence was 1800 nM and the concentration of NaCl(aq) was 200 mM was prepared, maintained at 95°C for 5 minutes in a thermal cycler, and then annealed from 95°C to 4°C at a rate of - 0.5 °C/30 seconds. 16.7 μL of each of the S1H1, S2H2, S3H1, S5H2, S6H1 and S7H2 solutions having a NaCl(aq) concentration of 200 mM and a sequence concentration of 1800 nM prepared as described above was mixed, maintained at 55°C for 5 minutes in a thermal cycler, and then annealed from 55°C to 4°C at a rate of -0.1 °C/144 seconds. Thereby, 100 μL of an H-DNA solution in which the final concentration of the structure was 300 nM and the final concentration of NaCl(aq) was 200 mM was completed.

1-5. Synthesis of Y-type DNA (Y-DNA) structure

As a DNA nanostructure comprising one pair of H1 and H2 motifs for comparison of the flexibility of DNA nanostructures, a Y-DNA structure (comprising H1 and H2 motifs at only two branches, among three double-stranded branches) was synthesized. Specifically, sequences in which BHQ2 was substituted at the 3' end of the S1H1 sequence and a Cy5 fluorophore was substituted at the 5' end of the S9H2 sequence were used. 33.3 μL of each of solutions in which the concentration of the S2, S9H2 or S1H1 sequence was 900 nM and the concentration of NaCl(aq) was 200 mM was prepared, maintained at 95°C for 5 minutes in a thermal cycler, and then annealed from 95°C to 4°C at a rate of -0.5 °C/30 seconds. 33.3 μL of each of the S1H1 and S9H2 solutions having a NaCl(aq) concentration of 200 mM and a sequence concentration of 900 nM prepared as described above was mixed, maintained at 43.5°C for 5 minutes in a thermal cycler, and then annealed from 43.5°C to 4°C at a rate of -0.5 °C/30 seconds, thereby completing 100 μL of a Y-DNA solution in which the final concentration of the structure was 300 nM and the final concentration of NaCl(aq) was 200 mM.

1-6. Synthesis of 2-arm T-DNA structure

As a DNA nanostructure comprising one pair of H1 and H2 motifs for comparison of the flexibility of DNA nanostructures, a T-DNA structure (comprising H1 and H2 motifs at only two branches, among four double-stranded branches) was synthesized. Specifically, sequences in which BHQ2 was substituted at the 3' end of the S1H1 sequence and a Cy5 fluorophore was substituted at the 5' end of the S2 sequence were used. 25 μL of each of solutions in which the concentration of the S2, S3, S4H2 or S1H1 sequence was 1200 nM and the concentration of NaCl(aq) was 200 mM was prepared, maintained at 95°C for 5 minutes in a thermal cycler, and then annealed from 95°C to 4°C at a rate of -0.5 °C/30 seconds. 25 μL of each of the H1S1, S2, S3 and S4H2 solutions having a NaCl(aq) concentration of 200 mM and a sequence concentration of 1200 nM prepared as described above was mixed, maintained at 43.5°C for 5 minutes in a thermal cycler, and then annealed from 43.5°C to 4°C at a rate of -0.5 °C/30 seconds. Thereby, 100 μL of a 2-arm T-DNA solution in which the final concentration of the structure was 300 nM and the final concentration of NaCl(aq) was 200 mM was completed.

The nucleotide sequences of the probe pairs, the structure nucleic acids and the target nucleic acid prepared and used in Example 1 are shown in the following table.

| List | | | Nucleotide sequence (5' ->3') | SEQ ID NO: |
|---|---|---|---|---|
| Probe pair (DNA) | | H1 | tcaacatc**ag tctgataagc ta**ccatgtgt aga**tagctta tcagact** (47 bp) | 20 |
| | | H2 | taagcta**tct acacatgg**ta gcttatcaga ct**ccatgtgt aga** (43bp) | 21 |
| Structure nucleic acid (DNA) | | S1 | cgaccgatga atagcggtca gatccgtacc tactcg (36bp) | 1 |
| | | S2 | cgagtaggta cggatctgcg tattgcgaac gactcg (36bp) | 2 |
| | | S3 | cgagtcgttc gcaatacggc tgtacgtatg gtctcg (36bp) | 3 |
| | | S4 | cgagaccata cgtacagcac cgctattcat cggtcg (36bp) | 4 |
| | | S5 | cgagaccata cgtacagcgc gatgcgcacg cgcacg (36bp) | 5 |
| | | S6 | cgtgcgcgtg cgcatcgctg cggtgccgtg tgcacg (36bp) | 6 |
| | | S7 | cgtgcacacg gcaccgcaac cgctattcat cggtcg (36bp) | 7 |
| | | S8 | cgagtaggta cggatctgac cgctattcat cggtcg (36bp) | 8 |
| | | S9 | cgagtcgttc gcaatacgac cgctattcat cggtcg (36bp) | 9 |

(continued)

| List | | Nucleotide sequence (5' ->3') | SEQ ID NO: |
|---|---|---|---|
| Probe/ structure strand | S1H1 | cgaccgatga atagcggtca gatccgtacc tactcgtcaa catc**agtctg ataagcta**cc atgtgtaga**t agcttatcag act** (83bp) | 10 |
| | S2H2 | cgagtaggta cggatctgcg tattgcgaac gactcgtaag cta**tctacac atgg**tagctt atcagact**cc atgtgtaga** (79bp) | 11 |
| | S3H1 | cgagtcgttc gcaatacggc tgtacgtatg gtctcgtcaa catc**agtctg ataagcta**cc atgtgtaga**t agcttatcag act** (83bp) | 12 |
| | S4H2 | cgagaccata cgtacagcac cgctattcat cggtcgtaag cta**tctacac atgg**tagctt atcagact**cc atgtgtaga** (79bp) | 13 |
| | S5H2 | cgagaccata cgtacagcgc gatgcgcacg cgcacgtaag cta**tctacac atgg**tagctt atcagact**cc atgtgtaga** (79bp) | 14 |
| | S6H1 | cgtgcgcgtg cgcatcgctg cggtgccgtg tgcacgtcaa catc**agtctg ataagcta**cc atgtgtaga**t agcttatcag act** (83bp) | 15 |
| | S7H2 | cgtgcacacg gcaccgcaac cgctattcat cggtcgtaag cta**tctacac atgg**tagctt atcagact**cc atgtgtaga** (79bp) | 16 |
| | S8H2 | cgagtaggta cggatctgac cgctattcat cggtcgtaag cta**tctacac atgg**tagctt atcagact**cc atgtgtaga** (79bp) | 17 |
| | S9H2 | cgagtcgttc gcaatacgac cgctattcat cggtcgtaag cta**tctacac atgg**tagctt atcagact**cc atgtgtaga** (79bp) | 18 |
| Target nucleic acid (RNA) | miR-21 | uagcuuauca gacugauguu ga (22bp) | 19 |
| *The probe is provided with a hairpin loop through hybridization of the sequences indicated in bold. | | | |

Example 2. Confirmation of synthesis of nanostructure

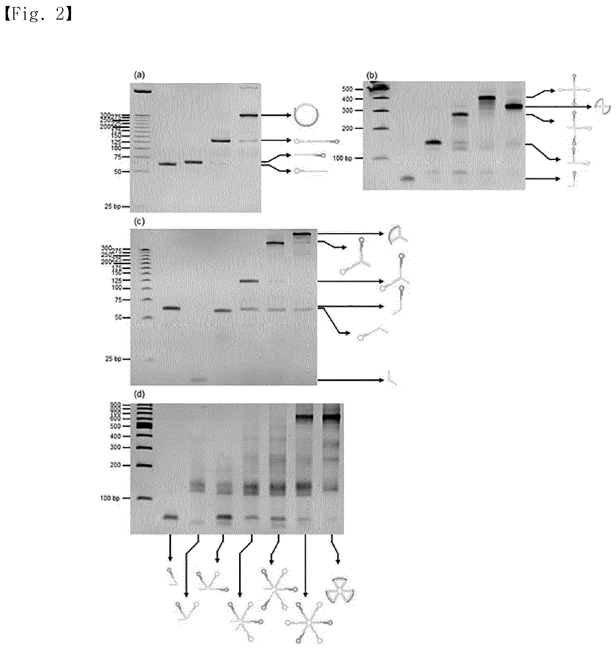

In order to confirm whether the D-DNA, T-DNA, H-DNA and Y-DNA structures constructed in Example 1 were synthesized, 1× TBE 5% or 12% polyacrylamide gel electrophoresis (1× tris/borate/ EDTA polyacrylamide gel electrophoresis) was performed.
Based on the results thereof, as shown in FIG. 2, it was found that all of the D-DNA, T-DNA, H-DNA and Y-DNA structures were successfully synthesized (FIG. 2).

Example 3. Comparison of reactivity of separated probe system and probe system integrated into DNA nanostructure with target material

3-1. Separated H1 and H2

16.7 μL of a solution in which the concentration of NaCl(aq) was 50 mM and the concentration of the miR-21 sequence was 0 nM, 30 nM, 60 nM, 90 nM, 120 nM, 150 nM, 180 nM, 210 nM, 240 nM, 300 nM, 450 nM, or 600 nM was mixed with 16.7 μL of each of the separated 300 nM H1 and H2 solutions synthesized in Example 1, so that the final concentration of each of H1 and H2 was 100 nM, the final concentration of miR-21 was 0 nM, 10 nM, 20 nM, 30 nM, 40 nM, 50 nM, 60 nM, 70 nM, 80 nM, 100 nM, 150 nM, or 200 nM, and the final concentration of NaCl(aq) was 150 mM. Since it is possible to measure the unwinding of the hairpin loop of H1, which represents the reaction with miR-21, using the Cy5 fluorophore and the quencher, the Cy5 fluorophore was excited at a wavelength of 640 nm using a spectrometer at 37°C, and thus the intensity of the emission wavelength of 670 nm was measured at 3-minute intervals over 24 hours, whereby the time-dependent reaction with miR-21 at various concentrations was confirmed (FIG. 3e), and moreover, electrophoretic analysis before and after reaction of the probe depending on the concentration of miR-21 (FIG. 3a), the extent of reaction depending on the concentration of miR-21 24 hours after reaction (FIG. 3c), and the detection limit depending on the reaction time (FIG. 3d) were confirmed (FIG. 3).

3-2. D-DNA

16.7 μL of a 200 mM NaCl(aq) solution, 16.7 μL of a solution in which the concentration of the miR-21 sequence was 0 nM, 30 nM, 60 nM, 90 nM, 120 nM, 150 nM, 180 nM, 210 nM, 240 nM, 300 nM, 450 nM, or 600 nM, and 16.7 μL of the 300 nM D-DNA structure solution synthesized in Example 1 were mixed, so that the final concentration of the structure was 100 nM and the final concentration of NaCl(aq) was 150 mM. Thereafter, the Cy5 fluorophore was excited at a wavelength of 640 nm using a spectrometer at 37°C, and thus the intensity of the emission wavelength of 670 nm was measured at 3-minute intervals over 24 hours, whereby the time-dependent reaction with miR-21 at various concentrations was confirmed (FIG. 3f), and moreover, electrophoretic analysis before and after reaction of the probe depending on the concentration of miR-21 (FIG. 3a), the extent of reaction depending on the concentration of miR-21 24 hours after reaction (FIG. 3c), and the detection limit depending on the reaction time (FIG. 3d) were confirmed (FIG. 3).

3-3. T-DNA

8.3 μL of the 300 nM T-DNA structure solution synthesized in Example 1 was mixed with 16.7 μL of the miR-21 solution at various concentrations as in 2-1 above and 25 μL of a 200 mM NaCl(aq) solution, so that the final concentration of the structure was 50 nM and the final concentration of NaCl(aq) was 150 mM. Thereafter, the Cy5 fluorophore was excited at a wavelength of 640 nm using a spectrometer at 37°C and thus the intensity of the emission wavelength of 670 nm was measured at 3-minute intervals over 24 hours, whereby the time-dependent reaction with miR-21 at various concentrations was confirmed (FIG. 3g), and moreover, electrophoretic analysis before and after reaction of the probe depending on the concentration of miR-21 (FIG. 3a), the extent of reaction depending on the concentration of miR-21 24 hours after reaction (FIG. 3c), and the detection limit depending on the reaction time, calculated based on the guidelines of the Clinical and Laboratory Standards Institution (CLSI) (FIG. 3d), were confirmed (FIG. 3). Based on the results thereof, the γ structure was confirmed through electrophoresis.

3-4. H-DNA

5.5 μL of the 300 nM H-DNA structure solution synthesized in Example 1 was mixed with 16.7 μL of the miR-21 solution at various concentrations as in 2-1 above and 27.8 μL of a 200 mM NaCl(aq) solution, so that the final concentration of the structure was 33.3 nM and the final concentration of NaCl(aq) was 150 mM. Thereafter, the Cy5 fluorophore was excited at a wavelength of 640 nm using a spectrometer at 37°C and thus the intensity of the emission wavelength of 670 nm was measured at 3-minute intervals over 24 hours, whereby the time-dependent reaction with miR-21 at various concentrations was confirmed (FIG. 3h), and moreover, electrophoretic analysis before and after reaction of the probe depending on the concentration of miR-21 (FIG. 3a), the extent of reaction depending on the concentration of miR-21 24 hours after reaction (FIG. 3c), and the detection limit depending on the reaction time (FIG. 3d) were confirmed (FIG. 3).

3-5. Comparison of final reactivity of separated H1 and H2, D-DNA, T-DNA, and H-DNA and calculation of detection limit

Based on the results of e, f, g, and h of FIG. 3, in which the extent of unwinding of the hairpin loop of H1, that is, the extent of reaction of each probe system, was measured over 24 hours depending on a change in the fluorescence intensity of the fluorophore attached to each sequence, the extent of reaction of each structure sample with miR-21 at various concentrations measured at 24 hours exhibited high reactivity to low-concentration miR-21 in the order of H-DNA, T-DNA, D-DNA, and separated H1 and H2 when comparing the extents to which equilibrium had been reached under the assumption that the equilibrium fluorescence value of each structure using 200 nM miR-21 was 100% (FIG. 3c). In addition, based on the extent of reaction of each probe system, measured depending on the change in fluorescence intensity over 24 hours, the detection limit depending on the reaction time according to the guidelines of the Clinical and Laboratory Standards Institution (CLSI) was calculated using Equation 1 below, and showed that the detection limits of D-DNA, T-DNA and H-DNA, in which intramolecular reactions rather than intermolecular reactions occurred, were significantly reduced compared to the separated H1 and H2, and furthermore, the detection limit was shown to be lowest for H-DNA, followed by T-DNA and D-DNA, 12 hours after reaction (FIG. 3d). Moreover, for the H-DNA structure under H1 or H2 motif conditions at the same concentration (100 nM), the concentration of the H-DNA structure alone was low (33.3 nM), and the intermolecular collision frequency shown in FIG. 1b was relatively low, and thus the initial rate and reactivity were decreased compared to other probe systems.

[Equation 1]

$$\text{Fluorescence value of detection limit} = \text{fluorescence value of negative control} + 1.645*(\text{standard deviation of fluorescence value of negative control}) + 1.645*(\text{standard deviation of fluorescence value of low-concentration sample})$$

(negative control: the case in which the concentration of miR-21 is 0 nM; and low-concentration sample: the case in which the concentration of miR-21 is 10 nM)

3-6. Y-DNA

16.7 $\mu$L of the 300 nM Y-DNA structure solution synthesized in Example 1 was mixed with 16.7 $\mu$L of the miR-21 solution at various concentrations as in 2-1 above and 16.7 $\mu$L of a 200 mM NaCl(aq) solution, so that the final concentration of the structure was 100 nM and the final concentration of NaCl(aq) was 150 mM. Thereafter, the Cy5 fluorophore was excited at a wavelength of 640 nm using a spectrometer at 37°C and thus the intensity of the emission wavelength of 670 nm was measured at 3-minute intervals over 24 hours, whereby the time-dependent reaction with miR-21 at various concentrations was confirmed (FIG. 4e), and moreover, the initial reaction rate (FIG. 4b) and the detection limit depending on the reaction time (FIG. 4c) were confirmed.

3-7. 2-Arm T-DNA

16.7 $\mu$L of the 300 nM 2-arm T-DNA structure solution synthesized in Example 1 was mixed with 16.7 $\mu$L of the miR-21 solution at various concentrations as in 2-1 above and 16.7 $\mu$L of a 200 mM NaCl(aq) solution, so that the final concentration of the structure was 100 nM and the final concentration of NaCl(aq) was 150 mM. Thereafter, the Cy5 fluorophore was excited at a wavelength of 640 nm using a spectrometer at 37°C and thus the intensity of the emission wavelength of 670 nm was measured at 3-minute intervals over 24 hours, whereby the time-dependent reaction with miR-21 at various concentrations was confirmed (FIG. 4f), and moreover, the initial reaction rate (FIG. 4b) and the detection limit depending on the reaction time (FIG. 4c) were confirmed.

3-8. Calculation of detection limits and comparison of initial reaction rates

Based on the results of d, e, and f of FIG. 4, in which the extent of unwinding of the hairpin loop of H1, that is, the extent of reaction of each probe system, was measured over 24 hours depending on the change in the fluorescence intensity of the fluorophore attached to each sequence, the equation for the extent of reaction depending on the time was derived by regressing the graph of each structure at the miR-21 concentration to the nearest S-shaped Weibull model, and the slope of the graph 1 hour after the start of the reaction, that is, the reaction rate, was calculated using the same. As a result, with regard to the initial reaction rate, in which the reaction rate of each probe system 1 hour after the start of the reaction was compared depending on the concentration of miR-21, the reaction rate was found to be fastest for 2-arm T-DNA, followed by Y-DNA and D-DNA, at miR-21 concentrations of 0 nM to 40 nM, and the reaction reached saturation and thus the reaction rate started to decrease in the order of 2-arm T-DNA, Y-DNA, and D-DNA at miR-21 concentrations of 50 nM or higher (FIG. 4b). In addition, based on the results of measurement of the detection limit of each probe system depending on the reaction time according to the guidelines of the Clinical and Laboratory Standards Institution (CLSI), the detection limit was found to be lowest for 2-arm T-DNA, followed by Y-DNA and D-DNA, having great flexibility at the link site between H1 and H2 motifs (FIG. 4c).

**Example 4. Measurement of distance between probes in DNA nanostructure and calculation of collision frequency using oxDNA simulation program**

4-1. Calculation of distance between paired bases and collision frequency during formation of structure

For the formation of a DNA nanostructure and the analysis of the distance between paired bases in the structure, the configuration of D-DNA, T-DNA, and H-DNA, in which the H1 motif of each structure was bound to miR-21, was analyzed through a DNA nucleic acid simulation program using the parameters shown in FIG. 5. Specifically, in order to form each structure on the program, the second base pair from the end of the sequences complementarily binding to each other in each structure was bound through a "trap" function. Also, only in the H-DNA structure, which is relatively complex, the mutual trap function was additionally applied to the third base pair from the end thereof (mutual trap: ability to arbitrarily shorten the distance between two different bases on the coordinates, which is typically used to efficiently form a double bond between sequences).

4-2. Analysis of distance between paired bases in structure

In order to measure the distance during intramolecular reaction between H1 and H2 motifs integrated into the DNA nanostructure, the distance between the eighth base from the 3' end of the H1 motif, which was bound to miR-21, corresponding to the actual reaction start site in 4-1 above, and the first base from the 5' end of the H2 motif (base pair at which complementary binding between H1 and H2 begins) was measured through sequence-based molecular dynamic (MD) simulation, and the distances between H1 and H2 motifs in one, four, and nine combinations respectively for D-DNA, T-DNA and H-DNA were measured (FIG. 6).

4-3. Calculation of effective collision frequency of H1 and H2

Among the distance distributions between the paired bases at which complementary binding between the H1 and H2 motifs in the DNA nanostructure measured in 4-2 above begins, the collision frequency was calculated by measuring the time and the number of times the oxDNA program distance unit reached 1 (a distance sufficient for a valid reaction to occur between commonly used paired bases) (FIG. 7). To this end, the intramolecular collision frequency was calculated depending on the number of times the distance between the H1 and H2 motifs was 1 based on the distance between the motifs measured in FIG. 6.

**Example 5 Theoretical calculation of collision frequency of separated H1 and H2**

The collision frequency between separated H1 and H2 probes corresponding to the existing single-stranded diagnostic system was theoretically calculated using the Smoluchowski equation based on the diffusion reaction in an aqueous solution. Specifically, the collision frequency of separated H1 and H2 was calculated using part of Smoluchowski's coagulation model of a solute in a liquid phase, the number of particles dispersed through Brownian motion toward any one particle was considered, and according to Fick's law, the flux of one particle passing through all particles surrounding a central particle having a radius r and eventually reaching the central particle, that is, the collision frequency with the central particle, may be represented by Equation 2 below.

[Equation 2]

$$F = 4\pi R D \upsilon_0$$

(F: flux; D: diffusion coefficient (diffusivity); R: distance between the centers of two particles; and u0: bulk concentration)

Here, assuming that the colliding particles have the same radius, the diffusion coefficient may be represented by Equation 3 below using the Stokes-Einstein equation.

[Equation 3]

$$D = 2K_B T / 3\pi\eta R$$

(kB: Boltzmann constant; and $\eta$: viscosity of the solution)

Substituting Equation 3 into Equation 2 and assuming that the viscosity of the solution is 759.873 $\mu$Pa·s according to the experimental conditions, the collision frequency of separated H1 and H2 is calculated to be 2710 $s^{-1}$. When comparing this value with the collision frequency in the intramolecular reaction of H1 and H2 motifs of each DNA nanostructure inferred through the oxDNA simulation analysis of Example 4, the intermolecular collision frequency of the separated H1 and H2 occurred twice for one miR-21, as shown in FIG. 1a, but the collision frequency of the intramolecular reaction that occurred once during the reaction of D-DNA, T-DNA, and H-DNA was found to be a minimum of at least 5 times and a maximum of at least 500 times as large as the collision frequency of the intermolecular reaction of the separated H1 and H2. Thereby, compared to the separated H1 and H2, the reactivity of the structure probe system of the present invention in which H1 and H2 motifs were integrated into the DNA nanostructure was confirmed to be high (FIG. 8). It can be found that the increased diagnostic sensitivity is due to the integrated probe sequence in the structure.

H1 and H2, which are two motifs having a hairpin loop, are capable of detecting miR-21 through an isothermal strand displacement reaction, and these two motifs are involved in separation of miR-21 from the detection probes and recycling thereof, which enables amplification of the detection signal. In this procedure, the intermolecular reaction occurred twice between the probes and miR-21 (FIG. 1a). The intermolecular reaction 2 of the separated H1 and H2 was converted into a more immediate and faster intramolecular reaction by integrating the two motifs, H1 and H2, into a double-stranded structure (FIG. 1b). Due to the integration of the separated H1 and H2 into the DNA nanostructure, the collision frequency between the motifs increased, thereby increasing the reactivity of the probe system (FIG. 1c).

[Industrial Applicability]

The nucleic acid nanostructure of the present invention enables some intermolecular collision/reaction steps due to simple diffusion to be converted into a fast reaction inside the nucleic acid structure through localized integration of probe sequences, so the signal amplification mechanism in the nucleic acid structure can be rapidly activated through structural flexibility, thereby increasing the ability to detect the target nucleic acid and improving detection efficiency, ultimately effectively lowering the final detection limit for the target nucleic acid.

Although specific embodiments of the present invention have been disclosed in detail as described above, it will be obvious to those of ordinary skill in the art that the description is merely of preferable exemplary embodiments, and is not to be construed as limiting the scope of the present invention. Therefore, the substantial scope of the present invention will be defined by the appended claims and equivalents thereof.

[National R&D project supporting this invention]
[Project identification number] 1711090207
[Project number] 2019R1A2C2002390
[Name of department] Ministry of Science and ICT
[Name of project management (specialized) institution] National Research Foundation of Korea
[Research project name] (Type 1-2) Mid-level research (within an average annual research cost of 100 to 200 million won) 1/3
[Research title] Research and development of genetically modified sequencing method using nanobarcoding technology of novel topological modification
[Contribution rate] 50/100
[Name of project execution institution] Sungkyunkwan University (Natural Science Campus)
[Research period] 2019.03.01 ~ 2020.02.29
[National R&D project supporting this invention]
[Project identification number] 465027898
[Project number] HI16C1984
[Name of department] Ministry of Health and Welfare
[Name of project management (specialized) institution] Korea Health Industry Development Institute
[Research project name] Post-genome multi-ministerial genome project 4/4
[Research title] Research and development of ultrahigh-speed real-time multi-diagnosis technology for human-derived lung cancer full-cycle gene biomarkers
[Contribution rate] 30/100
[Name of project execution institution] Sungkyunkwan University
[Research period] 2019.01.01 ~ 2019.05.23
[National R&D project supporting this invention]

[Project identification number] 1345277212
[Project number] 2017R1D1A1B03027897
[Name of department] Ministry of Education
[Name of project management (specialized) institution] National Research Foundation of Korea
[Research project name] Basic research (1-3 years) 3/4
[Research title] Research on production of artificial organelles capable of recognizing cancer cell-specific MicroRNA networks and artificial induction of apoptosis
[Contribution rate] 10/100
[Name of project execution institution] Sungkyunkwan University
[Research period] 2018.09.01 ~ 2019.06.30
[National R&D project supporting this invention]
[Project identification number] 1345286025
[Project number] 2016R1D1A1B03931270
[Name of department] Ministry of Education
[Name of project management (specialized) institution] National Research Foundation of Korea
[Research project name] 2016 2nd half of basic science and engineering (basic research) (1~3 years) 3/4
[Research title] Production of artificial organelles capable of recognizing cancer-cell-specific MicroRNA networks and new nanocomposite drug design driven by self-luminous light-heat chain reaction and prostate cancer treatment research thereof
[Contribution rate] 10/100
[Name of project execution institution] Sungkyunkwan University
[Research period] 2018.09.01 ~ 2019.06.30

[Sequence List Free Text]

An electronic file is attached.

## Claims

1. A nucleic acid nanostructure, in which probe-structure strands are hybridized with each other through complementary binding between structure nucleic acids,
   wherein each of the probe structure strands is formed by linking a probe pair, having a hairpin loop and comprising a nucleotide sequence specifically binding to a target nucleic acid, with the structure nucleic acids comprising sequences complementary to each other.

2.   The nucleic acid nanostructure according to claim 1, wherein probes of the probe pair comprise sequences complementary to each other.

3.   The nucleic acid nanostructure according to claim 1, wherein a probe is linked to a 3' end of the structure nucleic acid.

4.   The nucleic acid nanostructure according to claim 1, selected from the group consisting of:

(i) a nucleic acid nanostructure comprising one pair of probes;
(ii) a nucleic acid nanostructure comprising two pairs of probes; and
(iii) a nucleic acid nanostructure comprising three pairs of probes.

5.   The nucleic acid nanostructure according to claim 4, wherein the nucleic acid nanostructure comprising one pair of probes is a dimer-type (D-type) structure comprising respective single-stranded probes of a probe pair at both ends of a straight line,

wherein the dimer-type structure is consisted of a first probe-first structure strand and a second probe-second structure strand, and
wherein the first structure strand and the second structure strand, comprising complementary sequences, are complementarily bound to each other.

6.   The nucleic acid nanostructure according to claim 4, wherein the nucleic acid nanostructure comprising one pair of probes is a Y-type structure comprising respective single-stranded probes of a probe pair at ends of two branches, among three double-stranded branches,

wherein the Y-type structure is consisted of a first probe-first structure strand, a second structure strand, and a second probe-third structure strand,
wherein the first structure nucleic acid comprises a sequence complementary to a partial sequence of the second structure nucleic acid and a sequence complementary to a partial sequence of the third structure nucleic acid, the second structure nucleic acid comprises a sequence complementary to a partial sequence of the first structure nucleic acid and a sequence complementary to a partial sequence of the third structure nucleic acid, and the third structure nucleic acid comprises a sequence complementary to a partial sequence of the first structure nucleic acid and a sequence complementary to a partial sequence of the second structure nucleic acid, and
wherein the first probe-first structure strand, the second structure strand, and the second probe-third structure strand are complementarily bound to each other.

7.   The nucleic acid nanostructure according to claim 4, wherein the nucleic acid nanostructure comprising one pair of probes is a 2-arm tetramer structure comprising respective single-stranded probes of a probe pair at ends of two branches, among four double-stranded branches forming a cross shape,

wherein the 2-arm tetramer structure is consisted of a first probe-first structure strand, a second structure strand, a third structure strand, and a second probe-fourth structure strand,
wherein the first structure nucleic acid comprises a sequence complementary to a partial sequence of the second structure nucleic acid and a sequence complementary to a partial sequence of the fourth structure nucleic acid, the second structure nucleic acid comprises a sequence complementary to a partial sequence of the first structure nucleic acid and a sequence complementary to a partial sequence of the third structure nucleic acid, the third structure nucleic acid comprises a sequence complementary to a partial sequence of the first structure nucleic acid and a sequence complementary to a partial sequence of the fourth structure nucleic acid, and the fourth structure nucleic acid comprises a sequence complementary to a partial sequence of the third structure nucleic acid and a sequence complementary to a partial sequence of the first structure nucleic acid, and
wherein the first probe-first structure strand, the second structure strand, the third structure strand, and the second probe-fourth structure strand are complementarily bound to each other.

8.   The nucleic acid nanostructure according to claim 4, wherein the nucleic acid nanostructure comprising two pairs of probes is a tetramer-type (T-type) structure comprising single-stranded probes of each of two probe pairs at ends of four double-stranded branches,

wherein the tetramer-type structure is consisted of a first probe-first structure strand, a second probe-second structure strand, a first probe-third structure strand, and a second probe-fourth structure strand,
wherein the first structure nucleic acid comprises a sequence complementary to a partial sequence of the second structure nucleic acid and a sequence complementary to a partial sequence of the fourth structure nucleic acid, the second structure nucleic acid comprises a sequence complementary to a partial sequence of the first structure nucleic acid and a sequence complementary to a partial sequence of the third structure nucleic acid, the third structure nucleic acid comprises a sequence complementary to a partial sequence of the second structure nucleic acid and a sequence complementary to a partial sequence of the fourth structure nucleic acid, and the fourth structure nucleic acid comprises a sequence complementary to a partial sequence of the first structure nucleic acid and a sequence complementary to a partial sequence of the third structure nucleic acid, and wherein the first probe-first structure strand, the second probe-second structure strand, the first probe-third structure strand, and the second probe-fourth structure strand are complementarily bound to each other.

9. The nucleic acid nanostructure according to claim 3, wherein the nucleic acid nanostructure comprising three pairs of probes is a hexamer-type (H-type) structure comprising single-stranded probes of each of probe pairs alternating at ends of six double-stranded branches, The nucleic acid nanostructure according to claim 4, wherein the nucleic acid nanostructure comprising three pairs of probes is a hexamer-type (H-type) structure comprising single-stranded probes of each of probe pairs alternating at ends of six double-stranded branches,

wherein the hexamer-type (H-type) structure is consisted of a first probe-first structure strand, a second probe-second structure strand, a first probe-third structure strand, a second probe-fourth structure strand, a first probe-fifth structure strand, and a second probe-sixth structure strand,
wherein the first structure nucleic acid comprises a sequence complementary to the second structure nucleic acid and a sequence complementary to a partial sequence of the sixth structure nucleic acid, the second structure nucleic acid comprises a sequence complementary to a partial sequence of the first structure nucleic acid and a sequence complementary to a partial sequence of the third structure nucleic acid, the third structure nucleic acid comprises a sequence complementary to a partial sequence of the second structure nucleic acid and a sequence complementary to a partial sequence of the fourth structure nucleic acid, the fourth structure nucleic acid comprises a sequence complementary to a partial sequence of the third structure nucleic acid and a sequence complementary to a partial sequence of the fifth structure nucleic acid, the fifth structure nucleic acid comprises a sequence complementary to a partial sequence of the fourth structure nucleic acid and a sequence complementary to a partial sequence of the sixth structure nucleic acid, and the sixth structure nucleic acid comprises a sequence complementary to a partial sequence of the first structure nucleic acid and a sequence complementary to a partial sequence of the fifth structure nucleic acid, and
wherein the first probe-first structure strand, the second probe-second structure strand, the first probe-third structure strand, the second probe-fourth structure strand, the first probe-fifth structure strand, and the second probe-sixth structure strand are complementarily bound to each other.

10. The nucleic acid nanostructure according to claim 1, wherein a nucleic acid is DNA, RNA, or DNA and RNA.

11. The nucleic acid nanostructure according to claim 1, comprising a quencher at a 5' end or a 3' end thereof and a fluorophore at the 3' end or the 5' end thereof.

12. A composition for detecting a target nucleic acid comprising the nucleic acid nanostructure according to claim 1.

13. A diagnostic kit comprising the composition for detecting the nucleic acid according to claim 12.

14. A method of preparing a nucleic acid nanostructure, comprising:

(a) constructing probe-structure strands, in which a probe pair, having hairpin loop and comprising a nucleotide sequence specifically binding to a target nucleic acid, is respectively linked with structure nucleic acids comprising sequences complementary to each other; and
(b) annealing the probe-structure strands to obtain a nucleic acid nanostructure.

15. The method according to claim 14, wherein, in step (a), wherein the probe-structure strands are constructed by linking probes and the structure nucleic acids through annealing from 95°C to 4°C at a rate of - 5°C/30 seconds.

16. The method according to claim 14, wherein step (b) is performed by setting each of the probe-structure strands to a concentration of 500 to 700 nM, maintaining at 40 to 45°C for 3 to 10 minutes, and then annealing to 4°C at a rate of -0.5 °C/30 seconds.

17. The method according to claim 14, wherein step (b) is performed by setting each of the probe-structure strands to a concentration of 800 to 1000 nM, maintaining at 40 to 45°C for 3 to 10 minutes, and then annealing to 4°C at a rate of -0.5 °C/30 seconds.

18. The method according to claim 14, wherein step (b) is performed by setting each of the probe-structure strands to a concentration of 1100 to 1300 nM, maintaining at 40 to 45°C for 3 to 10 minutes, and then annealing to 4°C at a rate of -0.5 °C/30 seconds.

19. The method according to claim 14, wherein step (b) is performed by setting each of the probe-structure strands to a concentration of 1700 to 1900 nM, maintaining at 50 to 60°C for 3 to 10 minutes, and then annealing to 4°C at a rate of -0.1 °C/144 seconds.

20. A method of detecting a target nucleic acid using the nanostructure according to claim 1 or the composition for detecting the nucleic acid according to claim 12.

【Fig. 1】

EP 3 992 305 A1

【Fig. 2】

27

【Fig. 3】

【Fig. 4】

【Fig. 5】

| | Simulation type | Backend | Interaction type | Salt concentration (mM) | Time Interval (dT) | Simulation steps |
|---|---|---|---|---|---|---|
| Initial structure generation | Virtual move Monte Carlo (VMMC) | CPU | DNA2 | 1 | 0.003 | 1.00E+06 |
| Structure analysis | Molecular dynamics (MD) | CUDA | DNA2 | 0.15 | 0.001 | 1.00E+09 |

| | Temperature (°C) | Average sequence file | Used sequence dependent file | Simulation data print out Interval (Step) | External force (Mutual trap) |
|---|---|---|---|---|---|
| Initial structure generation | 25 (D-DNA) 43.5 (T-DNA) 43.5 (H-DNA) | N/A | N/A | 1000 | On |
| Structure analysis | 37 | Off | oxDNA2_seque nce_dependent_ parameters.txt | 10000 | Off |

【Fig. 6】

【Fig. 7】

【Fig. 8】

|  | Separated H1, H2 | D-DNA | T-DNA | H-DNA |
|---|---|---|---|---|
| H1, H2 motif concentration (nM) | 100 | 100 | 100 | 100 |
| DNA nanostructure concentration (nM) | N/A | 100 | 50 | 33.3 |
| Intermolecular collision frequency ($S^{-1}$) | 2.71E+03 | 1.36E+03 | 6.78E+02 | 4.53E+02 |
| Intramolecular collision frequency ($S^{-1}$) | N/A | 1.51E+04 | 2.92E+05 | 1.69E+06 |

## INTERNATIONAL SEARCH REPORT

| International application No. |
| --- |
| **PCT/KR2020/006934** |

| A. CLASSIFICATION OF SUBJECT MATTER |
| --- |
| *C12Q 1/6876(2018.01)i* |
| According to International Patent Classification (IPC) or to both national classification and IPC |

| B. FIELDS SEARCHED |
| --- |
| Minimum documentation searched (classification system followed by classification symbols)<br>C12Q 1/6876; C12N 15/00; C12N 15/115; C12Q 1/68; G01N 21/64 |
| Documentation searched other than minimum documentation to the extent that such documents are included in the fields searched<br>Korean utility models and applications for utility models: IPC as above<br>Japanese utility models and applications for utility models: IPC as above |
| Electronic data base consulted during the international search (name of data base and, where practicable, search terms used)<br>eKOMPASS (KIPO internal) & Keywords: hairpin probe, nucleic acid, nanostructure |

| C. DOCUMENTS CONSIDERED TO BE RELEVANT | | |
| --- | --- | --- |
| Category* | Citation of document, with indication, where appropriate, of the relevant passages | Relevant to claim No. |
| X | WEI, Q. et al. A DNA nanowire based localized catalytic hairpin assembly reaction for microRNA imaging in live cells. Chemical science. 2018, vol. 9, pages 7802-7808<br>See abstract; scheme 1; and pages 7802-7803 and 7806. | 1-20 |
| A | US 2014-0011189 A1 (MILLER, B. L. et al.) 09 January 2014<br>See abstract; paragraphs [0016]-[0017]; figures 2-3; and claim 1. | 1-20 |
| A | KR 10-2018-0072480 A (KOREA RESEARCH INSTITUTE OF BIOSCIENCE AND BIOTECHNOLOGY) 29 June 2018<br>See abstract; paragraph [0017]; and claim 1. | 1-20 |
| A | KR 10-2014-0024732 A (LG ELECTRONICS INC. et al.) 03 March 2014<br>See abstract; and claims 1-14. | 1-20 |
| A | KR 10-1991-0009926 A (MOLECULAR DIAGNOSTICS, INC.) 28 June 1991<br>See abstract; and claims 1-9. | 1-20 |

| ☐ Further documents are listed in the continuation of Box C. | ☒ See patent family annex. |
| --- | --- |

| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |
| Date of the actual completion of the international search<br>09 SEPTEMBER 2020 (09.09.2020) | Date of mailing of the international search report<br>**09 SEPTEMBER 2020 (09.09.2020)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

INTERNATIONAL SEARCH REPORT
Information on patent family members

International application No.

**PCT/KR2020/006934**

| Patent document cited in search report | Publication date | Patent family member | Publication date |
|---|---|---|---|
| US 2014-0011189 A1 | 09/01/2014 | CA 2511874 A1<br>US 2007-0059693 A1<br>WO 2004-061127 A2<br>WO 2004-061127 A3 | 22/07/2004<br>15/03/2007<br>22/07/2004<br>30/06/2005 |
| KR 10-2018-0072480 A | 29/06/2018 | WO 2018-117333 A1 | 28/06/2018 |
| KR 10-2014-0024732 A | 03/03/2014 | KR 10-1454511 B1<br>US 2014-0057256 A1 | 23/10/2014<br>27/02/2014 |
| KR 10-1991-0009926 A | 28/06/1991 | EP 0427073 A2<br>JP 4008293 A<br>US 5215899 A | 15/05/1991<br>13/01/1992<br>01/06/1993 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAIKI R.** *Science,* 1998, vol. 239, 487-91
- **HIGUCHI R.** *Nature Biotechnology,* 1993, vol. 11, 1026-1030
- **HELB D.** *J. Clin. Microbiol.,* 2010, vol. 48, 229-237
- **AVEYARD J.** *Chem. Commun.,* 2007, vol. 41, 4251-4253